# EUROPÄISCHE PATENTSCHRIFT

(11) **EP 2 922 497 B1**
(45) Veröffentlichungstag und Bekanntmachung des Hinweises auf die Patenterteilung: **25.10.2017**
(21) Anmeldenummer: 13798615.4
(22) Anmeldetag: 21.11.2013
(51) Int. Cl.: A61F 2/07, A61F 2/856

(54) **GEFASSIMPLANTAT MIT SEITENAST**
VASCULAR IMPLANT WITH SIDE BRANCH
IMPLANT VASCULAIRE COMPORTANT UNE RAMIFICATION LATÉRALE

(30) Priorität: 21.11.2012 DE 102012111225
(43) Veröffentlichungstag der Anmeldung: 30.09.2015
(73) Patentinhaber: JOTEC GmbH, 72379 Hechingen (DE)
(72) Erfinder: FISCHER, Heike, 40670 Meerbusch (DE); MERZ, Juergen, 72336 Balingen (DE)
(74) Vertreter: Witte, Weller & Partner Patentanwälte mbB
(86) Internationale Anmeldenummer: PCT/EP2013/074367
(87) Internationale Veröffentlichungsnummer: WO 2014/079919

(56) Entgegenhaltungen:
- EP-A1- 2 522 305
- US-A1- 2007 167 955
- US-A1- 2011 270 380

## Beschreibung

Die vorliegende Erfindung betrifft ein Gefäßimplantat zur Implantation in Blutgefäß eines Patienten, wobei das Gefäßimplantat von einem komprimierten Zustand in einen selbstexpandierten Zustand überführbar ist, mit einem hohlzylindrischen Grundkörper mit einem proximalen und einem distalen Ende, einem Grundkörperlumen und einer Längsrichtung, und mit einem Abschnitt, der in der Längsrichtung des Grundkörpers hintereinander angeordnete und senkrecht zur Längsrichtung jeweils mäanderförmig umlaufende jeweils einstückige Stentfedern und ein an den Stentfedern befestigtes und diese verbindendes Implantatmaterial aufweist, wobei die Stentfedern lediglich über das Implantatmaterial und nicht untereinander verbunden sind. Die mäanderförmig umlaufenden Stentfedern weisen abwechselnd zum proximalen und distalen Ende des Grundkörpers und parallel zu dessen Längsrichtung weisende Spitzbögen auf. Das Gefäßimplantat weist ferner zumindest einem vom Grundkörper abzweigenden hohlzylindrischen Seitenkörper mit einem Seitenkörperlumen und einem Seitenkörper-Implantatmaterial, wobei das Seitenkörperlumen mit dem Grundkörperlumen in Fluidverbindung steht.

Derartige so genannte verzweigte Gefäßimplantate sind im Stand der Technik bekannt. Solche Gefäßimplantate, die auch als endovaskuläre Gefäßstents/-stentgrafts bezeichnet werden, werden zur Behandlung von Aneurysmen in Arterien implantiert. Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysmas kann im fortgeschrittenen Stadium zu einer Ruptur der Arterie führen, mit der Folge, dass der Patient innerlich verblutet.

Zur Behandlung von Aneurysmen wird daher das betroffene Gefäß durch Implantation eines Stents/Stentgrafts stabilisiert, um eine Ruptur des Gefäßes zu vermeiden.

Die zur Behandlung von Aneurysmen verwendeten Stents/Stentgrafts bzw. Gefäßimplantate bestehen dabei im Allgemeinen aus einem röhrchenförmigen/hohlzylindrischen Metallrahmen, oder aus einzelnen, hintereinander angeordneten Metall-(Stent)federn, dessen bzw. deren Mantelfläche mit einer Textil- oder Polymerfolie abgedeckt ist, so dass sich ein hohlzylindrischer Körper ergibt. Zur Implantation wird dieser Stent/Stentgraft dann bspw. mittels einer diesen umgebenden und komprimierenden Hülle radial zusammengedrückt, so dass sich seine Querschnittsfläche deutlich verringert. Der Stent/Stentgraft wird dann mithilfe eines Einführsystems in den Bereich des Aneurysmas gebracht, wo der Stent freigesetzt wird. Aufgrund der Federwirkung des Metallrahmens bzw. der Metallfedern expandiert der Stent wieder in seine ursprüngliche Form und spannt dabei seine Mantelfläche auf, die sich proximal und distal von dem Aneurysma innen in dem Blutgefäß verklemmt. Auf diese Weise kann das Blut jetzt durch den Stent/Stentgraft fließen, wodurch eine weitere Belastung der Aussackung verhindert wird.

Die Expansion des Metallrahmens bzw. der Metallelemente kann dabei einerseits durch die Verwendung von selbstexpandierendem Metall, wie bspw. Nitinol, bewirkt werden, oder aber durch den Einsatz eines Dilatationsballons, der von innen in den Metallrahmen eingeführt wird und dessen Dilatation auch der Metallrahmen/die Metallelemente expandiert wird/werden.

Oftmals zweigen an der Stelle des Gefäßes, an welcher ein Stent/Stentgraft bzw. ein solches Gefäßimplantat eingeführt werden soll, seitlich Blutgefäße ab, weshalb bei Einbringung des Gefäßimplantats an solchen Abzweigungsstellen im Gefäß die Gefahr besteht, dass diese Seitengefäße durch das Gefäßimplantat im Hauptgefäß bzw. durch das ggf. blutdichte Implantatmaterial von der Blutzufuhr abgeschnitten werden. Daher weisen Gefäßimplantate in diesen Bereichen oftmals Öffnungen, so genannte "Fenestrierungen", im Mantel- bzw. Implantatmaterial auf, um das durch das Gefäßimplantat fließende Blut durch diese Öffnungen und in der Regel durch in diesen Öffnungen separat angebrachte, in die Seitengefäße geführte Seitenäste des Gefäßimplants, auch in die vom Gefäß abzweigenden Seiten-Gefäße zu lenken. Dadurch wird auch eine Blutversorgung der Körperbereiche, die von dem Seiten-Gefäß versorgt werden, gewährleistet.

In vielen Fällen weisen die an solchen abzweigenden Bereichen einzubringenden Gefäßimplantate bzw. Stents/Stentgrafts nicht nur Fenestrierungen, sondern vom Gefäßimplantatgrundkörper abzweigende Seitenäste auf, die im Aneurysmasack oberhalb des abzweigenden Gefäßes freigesetzt werden und bspw. als Landungszone für einen weiteren Stentgraft dienen, der zur Überbrückung des Aneurysmas in den Seitenast und das abzweigende Gefäß implantiert wird. Dadurch wird zusätzlich sichergestellt, dass auch die Seiten-Gefäße mit Blut versorgt werden.

Kritische Stellen im Gefäßsystem des Menschen stellen bspw. der Bereich der Aorten-Bifurkation, sowie die Aufzweigung der Arteriae iliacae communes in die Arteriae iliacae externa und die Arteriae iliacae interna dar. Bei beiden Abzweigungen ist es meist wichtig, die Versorgung der sich communes abzweigenden Gefäße zu gewährleisten, wenn die Aorta bzw. der Arteriae iliacae in diesem Bereich bspw. aufgrund eines Aneurysmas behandelt werden muss. Im Stand der Technik werden gegenwärtig zur Behandlung von abdominalen Aneurysmen üblicherweise Stents bzw. Stentgrafts (zusammen auch: "Gefäßimplantate") eingesetzt, die aus einem Grundkörper mit zwei sich nach distal erstreckenden Beinchen bestehen, welche zur Platzierung in den Arteriae iliacae communes vorgesehen sind. Ein solches Gefäßimplantat wird aufgrund ihrer Form und Platzierung auch als Y-Prothese bezeichnet.

Bei vielen dieser Implantate wird das zweite Beinchen, das auch als kontralaterales Bein bezeichnet wird, separat zum Grundkörper und erst nach dessen Platzierung eingeführt, um insgesamt ein einfacheres Platzieren der Prothese zu ermöglichen. Entsprechend wird bei diesen Implantat zuerst der Grundkörper (oder "main body") über einen - in der Regel - transfemoralen Zugang oberhalb der Aortenbifurkation abgesetzt. In einem zweiten Schritt wird dann ein weiteres Implantat, also das kontralaterale Bein, an den Grundkörper angedockt und die Bifurkationsprothese dadurch komplettiert.

Die EP 2 522 305 A1 offenbart eine Gefäßprothese mit einem Seitenast, der an den Hauptkörper winklig angenäht oder anderweitig winklig fixiert wird.

Die US 2011/0270380 A1 offenbart eine Gefäßprothese mit einem separaten Kupplungselement für einen Seitenast, welches den Seitenast in einem rechten Winkel aufstellt.

Die gegenwärtig im Stand der Technik bekannten und erhältlichen Gefäßimplantate mit Seitenästen sind üblicherweise entweder durch zwischen den Stentfedern angesetzte Seitenäste oder aber durch zwischen die Spitzbögen gesetzte Seitenäste bewirkt. Nachteilig bei den im Stand der Technik bekannten, Seitenäste tragenden Gefäßimplantaten ist, dass diese Seitenäste aufweisenden Gefäßimplantate im Bereich der Abzweigung meist eine Materialanhäufung besitzen, wie bspw. einen Nitinolring im Seitenast, über welchen der Seitenast nach der Expansion geöffnet wird, oder wie bspw. zusätzlich eingenähte Implantatmaterial-Geweberinge, was wiederum größere Einführsystemen notwendig macht und in engen Gefäßen zu einer schwierigen Handhabung dieser Gefäßimplantate führen kann. Die Seitenäste sind darüber hinaus auch sehr instabil und dadurch schwierig in ihrer Handhabung, wenn sie einmal ins Gefäß eingebracht sind.

Aufgabe der vorliegenden Erfindung ist es daher, ein Gefäßimplantat bereitzustellen, die einfach und stabil in das Hauptgefäß und in das Seitengefäß, und damit in die Abzweigung eingeführt werden kann, wodurch vorteilhafterweise eine Materialanhäufung vermieden und ein ungestörter Blutfluss erzielt wird.

Erfindungsgemäß wird diese Aufgabe gelöst durch eine Weiterbildung des eingangs genannten Gefäßimplantats gelöst, bei welcher der vom Grundkörper abzweigende zumindest eine Seitenkörper im selbstexpandierten Zustand in einem Winkel bezüglich der Längsrichtung des Grundkörpers ausgestellt ist, wobei der Winkel durch eine im Verhältnis zur Längsrichtung des Grundkörpers vorgeformte abgewinkelte Ausstellung zumindest eines Spitzbogens einer Stentfeder des Grundkörpers im selbstexpandierten Zustand bewirkt ist.

Ferner wird diese Aufgabe durch ein Verfahren zur Herstellung eines solchen Gefäßimplantats gelöst.

Die der Erfindung zugrundeliegende Aufgabe wird auf diese Weise vollkommen gelöst.

Mit der vorgeformten Ausstellung zumindest eines Spitzbogens einer der Stentfedern winklig im Verhältnis zur Längsachse des Grundkörpers wird erreicht, dass sich der Seitenast automatisch aufstellt, und zwar gerade aufgrund der Federkraft des zumindest einen ausgestellt vorgeformten Spitzbogens zumindest einer Stentfeder in der vorgegebenen Form. Dabei kann der Aufstellwinkel des zumindest einen Seitenkörpers durch den Herstellungsprozess vorgegeben werden. Auf diese Weise kann überflüssiges Material im Bereich des zumindest einen Seitenkörpers, mit Hilfe dessen bisher ein Aufstellen des Seitenkörpers bewirkt wurde, vermieden werden, und gleichzeitig wird das Einführen des überbrückenden Stentgrafts in das Seitengefäß deutlich erleichtert. Die Selbstausstellung des Seitenkörpers wird dadurch erreicht, dass das erfindungsgemäße Gefäßimplantat in einem komprimierten Zustand in das zu behandelnde Gefäß bzw. in den abzweigenden Bereich eingeführt wird, und nach Entfernen der Hülle bzw. des komprimierenden Mittels das selbstexpandierende Gefäßimplantat expandieren kann, wodurch sich auch der zumindest eine ausgestellte Spitzbogen aufstellt, und damit den Seitenkörper entfalten kann. Die übrigen Spitzbögen der zumindest einen Stentfeder verlaufen dabei parallel zur Längsachse des Grundkörpers des Gefäßimplantats. Sie stehen somit im Verhältnis zur Längsachse des Gefäßimplantats nicht winklig ab.

Vorliegend wird dabei unter einer "Stentfeder" wie eingangs diskutiert, jedes einstückige, ringförmige Element verstanden, das sich aufgrund dessen Materials komprimieren lässt, und sich nach Entfernung des Kompressionsdrucks wieder federartig expandieren kann. Unter "mäanderförmig" wird vorliegen jeder schlingen- bzw. schleifenförmige Verlauf" der Stentfeder bzw. des Stentdrahtes verstanden, wobei jede Stentfeder einstückig, d.h. aus einem mäanderförmig umlaufenden Stentfederring gebildet ist.

Eine "mäanderförmig umlaufende einstückige Stentfeder" ist in diesem Zusammenhang vorliegend ein sich federartig expandierendes und komprimierbares ringförmiges Stentelement, das einen wellenartigen Umlauf besitzt, wobei sich Wellenberg und Wellental, die eine Phase bilden, abwechseln.

Vorteilhafterweise ist dabei ein Spitzbogen jeweils aus zwei Schenkeln und einem zwischen den Schenkeln liegenden Scheitelpunkt bzw. Tiefstpunkt gebildet.

"Zumindest ein Spitzbogen" bedeutet vorliegend, dass der Seitenkörper durch die Ausstellung eines einzigen Spitzbogens einer Stentfeder aufgespannt wird, oder aber durch zwei oder mehr Spitzbögen. In einer bevorzugten Ausführungsform sind zwei Spitzbögen einer Stentfeder ausgestellt. Grundsätzlich bildet die Mehrzahl der mäanderförmig umlaufenden Spitzbögen die Stentfeder des Grundkörpers, und der bzw. die ausgestellte(n) Spitzbogen/Spitzbögen bildet die Abzweigungsstelle für den Seitenkörper.

Vorliegend wird dabei allgemein mit "proximal" diejenige Position, Richtung oder ein Abschnitt oder Ende einer Komponente des Gefäßimplantats bezeichnet, die/der am nächsten zum Herzen des zu behandelnden Patienten liegt.

Entsprechend wird vorliegend mit "distal" diejenige Position, Richtung oder ein Abschnitt oder Ende einer Komponente des erfindungsgemäßen Gefäßimplantats bezeichnet, die vom Herzen eines Patienten weiter/am weitesten entfernt ist/führt.

Entsprechend sind vorliegend die "proximale" und die "distale" Öffnung des Gefäßimplantats die Öffnungen, durch die hindurch der Blutfluss durch den hohlzylindrischen Körper der Gefäßimplantat gewährleistet wird: Wenn das erfindungsgemäße Gefäßimplantat in einem Blutgefäß wie beispielsweise der Aorta implantiert ist, fließt also das vom Herzen kommende Blut durch die proximale Öffnung des Gefäßimplantats, und verlässt das Gefäßimplantat durch deren distale Öffnungen.

Das Gefäßimplantat bzw. dessen hohlzylindrischer Grundköper kann dabei über seine Gesamtlänge hinweg einen einheitlichen Durchmesser, oder aber unterschiedliche Durchmesser aufweisen.

Definitionsgemäß sind die Stentfedern nicht direkt miteinander verbunden, und weisen untereinander keine verbindenden Schenkel oder Streben oder ähnliche Verbindungselemente auf. Die Stentfedern sind lediglich über das Implantatmaterial, an welches die Stentfedern angebracht sind, miteinander verbunden, wodurch eine "indirekte Verbindung" zwischen den Stentfedern geschaffen wird.

Vorliegend wird unter "Stent" jede Vorrichtung oder eine Struktur bezeichnet, die einer Prothese eine Expansionskraft und/oder eine stützende Funktion verleiht.

Der Ausdruck "Stentgraft" soll vorliegend - wie auch im Stand der Technik - eine Prothese bedeuten, die einen bzw. mehrere Stent (oder Stentfedern) sowie ein damit verbundenes Implantat("graft")-Material aufweist, das ein Lumen durch zumindest einen Abschnitt der Prothese bildet.

Gemäß einer weiteren Ausführungsform des erfindungsgemäßen Gefäßimplantats ist bevorzugt, wenn der Winkel, mit dem der zumindest eine Seitenkörper vom Grundkörper bezüglich der Längsrichtung des Grundkörpers ausgestellt ist, zwischen 20° und 90°, beträgt, 20° und 90° beträgt, und insbesondere zwischen 35° und 55°, und insbesondere Winkel im Bereich 40° bis 50°, und noch bevorzugter im Bereich zwischen 44° bis 46°, und vorzugsweise 45°. Es versteht sich, dass bei dem erfindungsgemäßen Gefäßimplantat auch mehrere Seitenkörper vorgesehen sein können, die mit jeweils unterschiedlichen Winkeln im Verhältnis zur Längsrichtung des Gefäßimplantats aufgestellt sind.

Vorliegend sind damit auch ausdrücklich sämtliche Ziffern mit umfasst, die zwischen diesen Bereichen liegen; dem Fachmann wird klar sein, dass auch Toleranzbereiche der genannten Bereiche, die im außerhalb, d.h. über oder unterhalb der angegebenen Bereiche liegen, mit inbegriffen sind, und die innerhalb von üblichen Fertigungs- und Messtoleranzen liegen.

Der Winkel, mit dem der zumindest eine Seitenkörper vom Grundkörper ausgestellt ist, ist dabei spezifisch auf das jeweils zu behandelnde Gefäß bzw. den zu behandelnden Gefäßabschnitt abgestimmt. Dieser kann vom behandelnden Arzt vorher genau definiert werden. Es versteht sich, dass sich gemäß einer weiteren Ausführungsform des erfindungsgemäßen Gefäßimplantats der jeweilige genaue Winkel von Gefäßimplantat zu Gefäßimplantat unterscheiden kann. Generell ist durch die Stentfeder mit dem zumindest einen winklig ausgestellten Spitzbogen die Lage des Seitenastes *in vivo* flexibler als bei einer Realisierung ohne Winkel. Die Gefahr, dass der Seitenast abknickt ist geringer.

Gemäß einer Weiterbildung einer Ausführungsform des erfindungsgemäßen Gefäßimplantats weist der zumindest eine Seitenkörper senkrecht zu seiner Seitenkörper-Längsrichtung einen Abschnitt mit zumindest einer mäanderförmig umlaufenden Seitenkörper-Stentfeder und einem an der Seitenkörper-Stentfeder und der abgewinkelt ausgestellten Stentfeder des Grundkörpers befestigten und diese verbindenden Seitenkörper-Implantatmaterial auf, wobei die zumindest eine Seitenkörper-Stentfeder und die abgewinkelt ausgestellte Stentfeder des Grundkörpers lediglich über das Seitenkörper-Implantatmaterial und nicht untereinander verbunden sind.

Diese Ausführungsform hat den Vorteil, dass auch der zumindest eine Seitenkörper des erfindungsgemäßen Gefäßimplantats einen gestenteten Abschnitt aufweist, der eine sichere Verankerung für einen zweiten Stentgraft gewährleistet, der zur Überbrückung des Aneurysmasackes bis in das abzweigende Gefäß führt.

Vorliegend wird dabei unter einem "gestenteten Abschnitt" jeder Abschnitt eines Gefäßimplantats verstanden, der zumindest einen Stent bzw. ein Stentfederelement in Verbindung mit einem Prothesematerial aufweist.

Im Gegensatz dazu wird vorliegend unter einem "ungestenteten" Abschnitt jeder Abschnitt eines Gefäßimplantats verstanden, der lediglich aus Prothesematerial gebildet ist.

Gemäß einer weiteren Ausführungsform des erfindungsgemäßen Gefäßimplantats kann der Grundkörper und/oder der zumindest eine Seitenkörper neben dem ersten gestenteten Abschnitt zumindest noch einen weiteren ungestenteten Abschnitt und/oder einen Abschnitt mit einem geflochtenen oder gelaserten Stent/Stentgraft aufweisen, wobei sich der zweite Abschnitt proximal oder distal an den gestenteten Abschnitt anschließt. Dabei bedeutet "ein weiterer ungestenteter Abschnitt", dass dieser als proximale oder distale Verlängerung des Grundkörperlumens bzw. des Seitenastes vorgesehen ist, wobei der gestentete oder der ungestentete Abschnitt entweder als zum Grundkörper separates Teil in vivo nach Implantation des Gefäßimplantats eingesetzt wird, oder aber fest mit dem Gefäßimplantat verbunden ist. Diese Ausführungsform hat den Vorteil, dass der Seitenast in das abzweigende Gefäß, bspw. die Iliaca interna, verlängert werden kann, um bspw. das Aneurysma vom Blutdruck auszuschließen. Der geflochtene oder gelaserte Stentgraft kann dadurch das Aneurysma im Seitengefäß und in der Landungszone der Iliaca interna abdichten.

Bei Einsatz eines ungestenteten Abschnitts kann dieser mit der Gefäßwand vernäht werden kann, was insbesondere bei Gefäßimplantaten vorteilhaft ist, die unverrückbar in dem Gefäß angebracht werden sollen.

Bei Vorsehen eines Abschnitts mit einem geflochtenen unterstützenden Stent kann der geflochtene Stent entweder in Verbindung mit einem Implantatmaterial eingesetzt werden, und von innen in das Implantatmaterial eingebracht sein, wodurch dieses durch die Expansion des Drahtgeflechts ebenfalls an die Wand des Gefäßes gedrückt und dort verankert wird. Gegebenenfalls kann auch das Drahtgeflecht mit dem Implantatmaterial vernäht sein.

Darüber hinaus kann der Grundkörper und/oder der zumindest eine Seitenkörper auch einen vom Implantatmaterial freien Stentabschnitt aufweisen, wobei dieser Stentabschnitt dann vorzugsweise aus einem Drahtgeflecht gebildet ist, und lediglich - auch - der Verankerung des Gefäßimplantats im Blutgefäß dient.

Gemäß einer Weiterbildung der erfindungsgemäßen Gefäßimplantat-Stentfeder ist bevorzugt, wenn zumindest drei Schenkel, die einen ersten Scheitelpunkt, einen auf dem ersten Scheitelpunkt in umlaufender Richtung folgenden ersten Tiefstpunkt, einen den ersten Tiefstpunkt in umlaufender Richtung folgenden zweiten Scheitelpunkt, und einen den zweiten Scheitelpunkt in umlaufender Richtung folgenden zweiten Tiefstpunkt verbinden, jeweils unterschiedliche Längen aufweisen.

Diese Ausführungsform hat den Vorteil, dass der Verlauf einer Stentfeder insgesamt asymmetrisch ist, wodurch bei dem Gefäßimplantat insgesamt eine Knickbildung vermieden werden kann. Die Stentfeder weist somit unregelmäßige "Amplituden" auf, d.h. die Spitzbögen - oder "Wellenberge"/"Wellentäler" - sind aufgrund der unterschiedlich langen Schenkel unterschiedlich hoch bzw. tief. Es versteht sich, dass eine Stentfeder insgesamt unterschiedliche Schenkel-Längen ihrer Spitzbögen aufweisen kann, oder aber eben lediglich drei oder mehr als drei unterschiedlich lange. Auch können die relativen Abstände zwischen einem Scheitelpunkt und einem Tiefspunkt oder zwischen allen Scheitelpunkten und Tiefstpunkten einer Stentfeder unterschiedlich sein.

Bei der genannten Ausführungsform wird damit ein asymmetrischer mäanderförmiger Verlauf der umlaufenden Stentfedern erreicht, bei welchem jeweils zwei aufeinanderfolgende Wellenberge unterschiedliche Höhen besitzen, und damit auch die jeweils dazwischen liegenden Wellentäler unterschiedliche Tiefen. Insgesamt folgt also in umlaufender Richtung abwechselnd auf einen höheren Wellenberg/Scheitelpunkt ein Wellenberg/Scheitelpunkt, der niedriger bzw. weniger hoch ist als der vorherige Wellenberg/Scheitelpunkt, und auf diesen Wellenberg/Scheitelpunkt wieder ein Wellenberg/Scheitelpunkt, der höher ist als der Wellenberg/Scheitelpunkt unmittelbar davor, usw., wobei zwischen den unterschiedlich hohen Wellenbergen/Scheitelpunkten jeweils unterschiedlich tiefe Wellentäler/Tiefstpunkte liegen: Auch bei den Wellentälern/Tiefstpunkten folgt in umlaufender Richtung abwechselnd auf ein tieferes Wellental/Tiefstpunkt wieder ein Wellental/Tiefstpunkt, das weniger tief ist als das in umlaufender Richtung unmittelbar vorher angeordnete Wellental/Tiefstpunkt, und auf dieses Wellental/Tiefstpunkt wieder ein Wellental/Tiefstpunkt, das tiefer ist als das davor. Anders gesprochen besitzt eine Stentfeder also Spitzbögen mit mindestens zwei oder drei unterschiedlich tiefen Tiefstpunkten, wobei sich die unterschiedliche Tiefen auf eine senkrecht zur Längsrichtung des hohlzylindrischen Grundkörpers umlaufende, gedachte Linie beziehen, welche die tiefsten Tiefstpunkte miteinander verbindet. Definitionsgemäß liegen damit weniger tiefe Tiefstpunkte nicht auf dieser gedachten Linie, und gleiches gilt wiederum für die Scheitelpunkte.

Gemäß einer weiteren Ausführungsform ist dabei zusätzlich noch bevorzugt, wenn zumindest zwei in Längsrichtung hintereinander angeordnete Stentfedern in Bezug auf die Längsrichtung des hohlzylindrischen Grundkörpers und in Bezug aufeinander derart angeordnet sind, dass die Tiefstpunkte der Spitzbögen einer ersten Stentfeder jeweils den Scheitelpunkten von Spitzbögen einer in Längsrichtung des Gefäßimplantats der ersten Stentfeder nachgeordneten zweiten Stentfeder in einem Abstand und in einer gedachten Linie, die parallel zur Längsrichtung des Gefäßimplantats ist, gegenüber liegen, und zwar derart, dass ein tiefster Tiefstpunkt eines Spitzbogens der ersten Stenfeder einem Scheitelpunkt der distal nachgeordneten zweiten Stentfeder in einem Abstand gegenüber liegt, wobei dieser Scheitelpunkt weniger hoch ist als ein höchster Scheitelpunkt der zweiten Stentfeder; entsprechend kommt damit bei dieser Ausführungsform immer auch ein weniger tiefer Tiefstpunkt - also ein Tiefstpunkt, der weniger tief ist als ein tiefster Tiefpunkt der ersten Stentfeder- in einem Abstand zu und gegenüber einem höchsten Scheitelpunkt der distal nachgeordneten zweiten Stentfeder zu liegen, welcher höchste Scheitelpunkt höher ist als ein weniger hoher Scheitelpunkt der zweiten Stentfeder.

Mit dieser Ausführungsform wird erreicht, dass zwischen den benachbart gegenüber liegenden Spitzbögen zwei verschiedene Biegeebenen vorliegen, wodurch das Gefäßimplantat deutlich flexibler wird, seine Längsstabilität aber beibehält. Gleichzeitig kann es ohne abzuknicken in mehrere Ebenen gebogen werden. Es versteht sich, dass andere Ausführungen der erfindungsgemäßen asymmetrischen Stentfeder auch zu mehr als zwei Biegeebenen führen können.

Zusätzlich zu den unterschiedlichen Schenkellängen verleiht auch diese Phasenanordnung mindestens zweier, vorzugsweise aller Stentfedern des Gefäßimplantats ein asymmetrisches Muster, das zur Vermeidung der Knickbildung im höchsten Maße beiträgt.

Entsprechend ist in einer anderen Ausführungsform des erfindungsgemäßen Gefäßimplantats vorgesehen, wenn zumindest zwei in Längsrichtung hintereinander angeordnete Stentfedern jeweils Schenkel mit unterschiedlichen Längen aufweisen zur Ausbildung von umlaufend aufeinanderfolgenden unterschiedlich hohen Spitzbögen, mit höheren und kürzeren Spitzbögen, und dass ein in proximale Richtung x weisender höherer Spitzbogen einer ersten Stentfeder einem in distale Richtung y weisenden kürzeren Spitzbogen einer proximal nachgeordneten zweiten Stentfeder in einem Abstand und in einer gedachten Linie gegenüber liegt, die parallel zur Längsrichtung des Gefäßimplantats ist, und/oder dass ein in proximale Richtung x weisender kürzerer Spitzbogen einer ersten Stentfeder einem in distale Richtung y weisenden längeren Spitzbogen einer in proximale Richtung x nachgeordneten zweiten Stentfeder in einem Abstand und in einer gedachten Linie gegenüber liegt, die parallel zur Längsrichtung des Gefäßimplantats ist.

Auch mit dieser Ausführung der einzelnen Stenfedern und deren Anordnung zueinander wird ein asymmetrischer Stentfederverlauf erreicht.

Aufgrund der unterschiedlich langen Schenkel ergeben sich unterschiedliche hohe Spitzbögen, wobei deren Höhe bezüglich einer gedachten Linie bestimmt ist, die in umlaufender Richtung der Stentfeder und senkrecht zur Längsrichtung des Gefäßimplantats durch den/die höchsten Scheitelpunkte der in proximale Richtung weisenden Spitzbögen verläuft. Aufgrund dieser Ausgestaltung und Definition liegen stets Scheitelpunkte vor, die unterhalb dieser gedachten Linie durch die höchsten Scheitelpunkte liegen, und damit Scheitelpunkte darstellen, die kürzer als die höchsten Scheitelpunkte sind. Analog gilt dies auch bezüglich der in distale Richtung weisenden Spitzbögen bzw. der Tiefstpunkte: auch hier ist eine gedachte Linie in umlaufender Richtung einer Stentfeder durch den/die höchsten Scheitelpunkt(e) der in distale Richtung weisenden Spitzbögen gezogen, so dass höhere und kürzere Scheitelpunkte der in distale Richtung weisenden Spitzbögen vorliegen.

Beispielhafte Höhen der unterschiedlichen Spitzbögen liegen bspw. im Bereich von 4 bis 18 mm, vorzugsweise ca .8 mm bis 14 mm für die höchsten Spitzbögen, also für die Spitzbögen, die höher sind als kürzere Spitzbögen, und von 4 bis 10 mm vorzugsweise 6 mm bis 8 mm, für die kürzeren Spitzbögen. Dabei wird dem Fachmann klar sein, dass bei einer Stentfeder einerseits Spitzbögen mit zumindest zwei oder drei oder vier oder mehr unterschiedlich hohen Spitzbögen vorliegen können. Bei Vorliegen dreier unterschiedlich hoher Spitzbögen weist eine Stentfeder also drei unterschiedlich Höhen für die Spitzbögen auf, also zumindest einen ersten höheren Spitzbogen, dessen Höhe am höchsten ist, zumindest einen zweiten Spitzbogen, dessen Höhe kürzer ist als die des ersten Spitzbogens, und einen dritten Spitzbogen, dessen Höhe wiederum kürzer als die des zweiten Spitzbogens ist, etc. Beispielhafte Höhen, die vorliegend lediglich exemplarischen Zwecken dienen und nicht limitierend sein sollen, sind bspw. 10 mm (höhere Spitzbögen) und 8 mm (kürzere Spitzbögen); 12 mm (höhere Spitzbögen) und 8 mm (kürzere Spitzbögen); 12 mm (höhere Spitzbögen) und 9 mm (kürzere Spitzbögen); 12 mm (höhere Spitzbögen) und 10 mm (kürzere Spitzbögen); 16 mm (höhere Spitzbögen) und 14 mm (kürzere Spitzbögen), 16 mm (höhere Spitzbögen) und 13 mm (kürzere Spitzbögen); 16 mm (höhere Spitzbögen), 12 mm (kürzere Spitzbögen) und 10 mm (noch kürzere Spitzbögen); 10 mm (höhere Spitzbögen) und 8 mm (kürzere Spitzbögen); 12 mm (höhere Spitzbögen), 10 mm (kürzere Spitzbögen) und 8 mm (noch kürzere Spitzbögen).

Vorliegend und durchgehend durch die Beschreibung soll dabei der Begriff "ca." bedeuten, dass bei den genannten Bereichs- und Zahlenangaben auch solche noch mit umfasst sein sollen, die aufgrund von Messunterschieden oder Toleranzen für den Fachmann noch mit umfasst sind, und die geeignet sind, die der Erfindung zu Grunde liegende Aufgabe zu lösen bzw. hierzu beizutragen.

Allgemein ist die Bildung von Knicken in einem Gefäßimplantat insbesondere in Bereichen, in denen Blutgefäße einen Bogenverlauf haben, kritisch, da es bei einer Knickbildung in solchen Bogen zu einem blockierten oder gestörten/verwirbelten oder verminderten bis gar keinen Blutfluss kommen kann.

Durch die erfindungsgemäßen Ausbildungen des Gefäßimplantats mit asymmetrischen Stentfedern wird dies erfolgreich verhindert.

Gemäß einer Ausführungsform des erfindungsgemäßen Gefäßimplantats ist bevorzugt, wenn das erfindungsgemäß selbstexpandierende Gefäßimplantat mindestens drei, in Längsrichtung hintereinander angeordnete Stentfedern aufweist, die nicht unmittelbar untereinander, sondern lediglich über das Implantatmaterial miteinander verbunden sind.

Gemäß einer weiteren Ausführungsform ist bevorzugt, wenn das erfindungsgemäße Gefäßimplantat zwischen drei und zehn, vorzugsweise, drei, vier, fünf, sechs, sieben, acht, neun oder zehn hintereinander angeordnete Stentfedern aufweist.

Die Anzahl der Stentfedern hängt dabei von der notwendigen Länge des einzusetzenden Gefäßimplantats ab, bzw. von den zu überbrückenden Gefäßdefekten eines Patienten.

Gemäß einer Weiterbildung des erfindungsgemäßen Gefäßimplantats sind die Stentfedern mit dem diese indirekt verbindenden Implantatmaterial verbunden, wobei sie vorzugsweise mit dem Implantatmaterial vernäht sind. Gleiches gilt auch für die Stentfedern des Seitenkörpers. Dabei können die Stentfedern entweder im Innenlumen des Gefäßimplantats, d.h. von innen an das Implantatmaterial genäht sein, oder aber außen auf das Implantatmaterial.

Als Nahtmaterial zum Vernähen der Stenfedern mit dem Implantatmaterial werden Nahtmaterialien verwendet, die im Gebiet der Chirurgie und dem Gebiet der Stentimplantatstechnik bereits regelmäßig eingesetzt werden, und solche Nahtmaterialien bestehen in der Regel aus Polyester, Polyurethan, Polytetrafluorethylen, Polyethylen, ultrahochmolekulares Polyethylen (UHMWPE), Polypropylen.

Gemäß einer bevorzugten Ausführungsform des erfindungsgemäßen Gefäßimplantats weist diese zwischen einem vier, d.h. eins, zwei, drei oder vier vom Grundkörper winklig ausgestellte Seitenäste auf.

Vorteilhafterweise können damit die erfindungsgemäßen Gefäßimplantate auch in Gefäßabschnitte eingebracht werden, an welchen mehrere Seitenblutgefäße abzweigen. Der Fachmann wird anhand des jeweiligen Patienten bzw. Gefäßsituation abschätzen können, wie viele Seitenäste für das einzusetzende Gefäßimplantat notwendig sein werden.

Es versteht sich, dass eine einzelne Stentfeder mehrere ausgestellte Spitzbögen aufweisen kann, wodurch auch mehrere ausgestellten Seitenäste gebildet werden können, oder aber die ausgestellten Spitzbögen sind an unterschiedlichen Stentfedern vorgesehen, je nach Lage der abgehenden Seiten-Blutgefäße.

Ferner betrifft die vorliegende Erfindung auch ein Verfahren zur Herstellung eines erfindungsgemäßen Gefäßimplantats, wobei das Verfahren die folgenden Schritte aufweist:
- Bereitstellen eines Formstücks, das auf einem zylinderförmigen Grundkörper basiert und das ein winklig vom zylinderförmigen Grundkörper abstehendes und mit dem Formstück verbundenes Formstückelement aufweist, welches eine abgerundete schräge Fläche aufweist, und wobei das Formstück ferner stiftförmige Elemente in der Position von Scheitelpunkten mäanderförmig umlaufender Stentfedern aufweist,
- Bereitstellen eines Stentfeder-bildenden Drahtes, vorzugsweise aus Nitinol,
- Aufwickeln des Stentfeder-bildenden Drahtes unter Zug auf dem Formstück und über die stiftförmigen Elemente, derart, dass mäanderförmig umlaufenden Stentfedern mit Spitzbögen um das Formstück gewickelt und auf dem Formstück fixiert werden, wobei auf dem winklig abstehenden Formstückelement der zumindest eine winklig ausgestellte Spitzbogen ausgebildet wird.

Die mit den erfindungsgemäßen Verfahrensschritten gebildete Stentfeder kann in weiteren, sich anschließenden Schritten in einem Wärmebad gesintert werden, wodurch sie dauerhaft ihre vorgegebene Form behält.

In wiederum daran anschließenden Schritten können dann die Drahtenden mit einem Crimpröhrchen verbunden werden, und die Oberfläche der so hergestellten Stentfeder anschließend elektropoliert werden.

Durch ein solches Ausformen der Stentfeder, mit Hilfe derer der Seitenkörper aufgestellt werden kann, wird sichergestellt, dass diese Stentfeder zumindest einen, vorzugsweise zwei Spitzbögen aufweist, der/die von dem senkrecht zur Längsrichtung des Gefäßimplantats verlaufenden Spitzbögen ausgestellt ist/sind, und zwar in einem Winkel, der in Abhängigkeit des winklig abstehenden Formstückelements, bzw. in Abhängigkeit des Winkels, der durch die schräge Fläche bezüglich des zylinderförmigen Grundkörpers gebildet ist, steht.

Der Winkel kann dabei so eingestellt sein, dass der Seitenast sich innerhalb des Aneurysmas in einer bevorzugten Weise aufstellt, und dadurch ggf. mittels eines zweiten Stentgrafts eine sichere Überbrückung des Aneurysmasacks in das abzweigende Gefäß ermöglicht wird. Durch das Aufstellen des Seitenasts wird eine gute Durchblutung durch diesen und den ggf. verlängernden Stentgraft in das abzweigende Gefäß erzielt.

Die Stentfeder mit dem winklig abstehenden Spitzbogen wird dabei vorzugsweise, wie die weiteren, bei dem erfindungsgemäßen Gefäßimplantat vorgesehenen Stentfedern, aus einem Shape-Memory-Metall, vorzugsweise Nitinol, auf dem Formstück gebildet und wird zur Fixierung ihrer expandierten Form einer Wärmebehandlung unterzogen.

Die mit den erfindungsgemäßen Verfahrensschritten gebildete Stentfeder, kann dann bei einem erfindungsgemäßen Gefäßimplantat als diejenige Stentfeder eingesetzt werden, über welche ein winklig abstehender Spitzbogen für den dann ebenfalls winklig abstehenden Seitenast bereitgestellt. Kurz gesagt wird die erfindungsgemäß hergestellte Stentfeder zusammen mit weiteren Stentfedern ohne winklig abstehende Spitzbögen in Abständen voneinander an ein Implantatmaterial fixiert, vorzugsweise angenäht, zur Ausbildung des hohlzylindrischen Grundkörpers. Auch der winklig abstehende Spitzbogen kann dann bspw. mit einer weiteren Stentfeder an ein Implantatmaterial fixiert, vorzugsweise angenäht werden, zur Ausbildung des Seitenastes.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in Bezug auf diese nachstehend näher beschrieben. Es zeigen:
- Fig. 1: eine beispielhafte Ausführungsform eines Gefäßimplantats des Standes der Technik mit Seitenkörper;
- Fig. 2: eine beispielhafte Ausführungsform des erfindungsgemäßen Gefäßimplantats mit Seitenkörper; **A**: in räumlicher seitlicher Draufsicht; **B**: in schematischer seitlicher Draufsicht;
- Fig. 3: eine weitere Ausführungsform des erfindungsgemäßen Gefäßimplantats, mit dem Grundkörper in unterschiedlichen gebogenen Positionen (**A** und **B**);
- Fig. 4: ein Formstück, mit Hilfe dessen die Stentfeder mit ausgestelltem Spitzbogen hergestellt wird (**A**); eine Stentfeder eines erfindungsgemäßen Gefäßimplantats, wie sie mit aufgestelltem Spitzbogen auf dem Formstück aus Fig. 4a gebildet wurde (**B**); und
- Fig. 5: eine ausschnittsweise und schematische Darstellung des Verlaufs zweier in Längsrichtung des Gefäßimplantats nachgeordneter Stentfedern, wie sie in einem Ausführungsbeispiel eines erfindungsgemäßen Gefäßimplantats verwirklicht werden können.

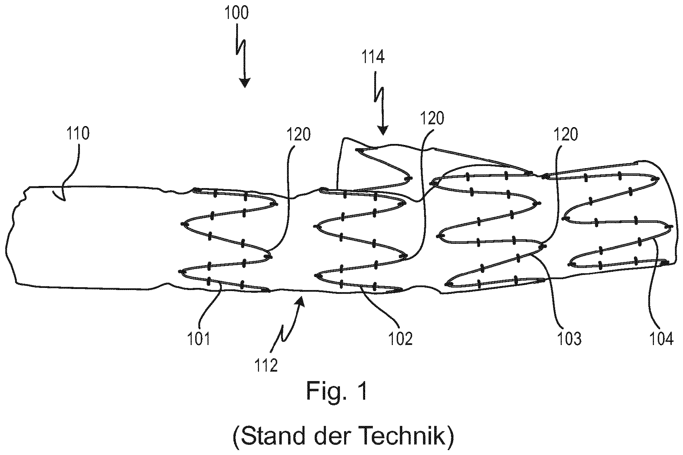

In Fig. 1 ist ein Gefäßimplantat 100 aus dem Stand der Technik dargestellt, die vier hintereinander angeordnete Stentfedern 101, 102, 103, 104 aufweist, wobei die Phasen der Stentfedern 101, 102, 103, 104 im Verhältnis zueinander symmetrisch verlaufen: Hier liegen sich also in Längsrichtung ein Scheitelpunkt 120 einer ersten Stentfeder 102 und ein Scheitelpunkt der distal darauf folgenden zweiten Stentfeder 103 exakt gegenüber, so dass die Stentfedern 101, 102, 103 und 104 auf einem Implantatmaterial 110 insgesamt ein symmetrisches Muster bilden. Das Gefäßimplantat 100 weist einen Grundkörper 112 und einen Seitenkörper 114 auf, welcher vom Grundköper 112 abzweigt und an diesen über das Implantatmaterial 110 angenäht ist. Aufgrund einer Materialanhäufung im Abzweigungsbereich steht der Seitenkörper 114 geringfügig vom Grundköper 112 ab.

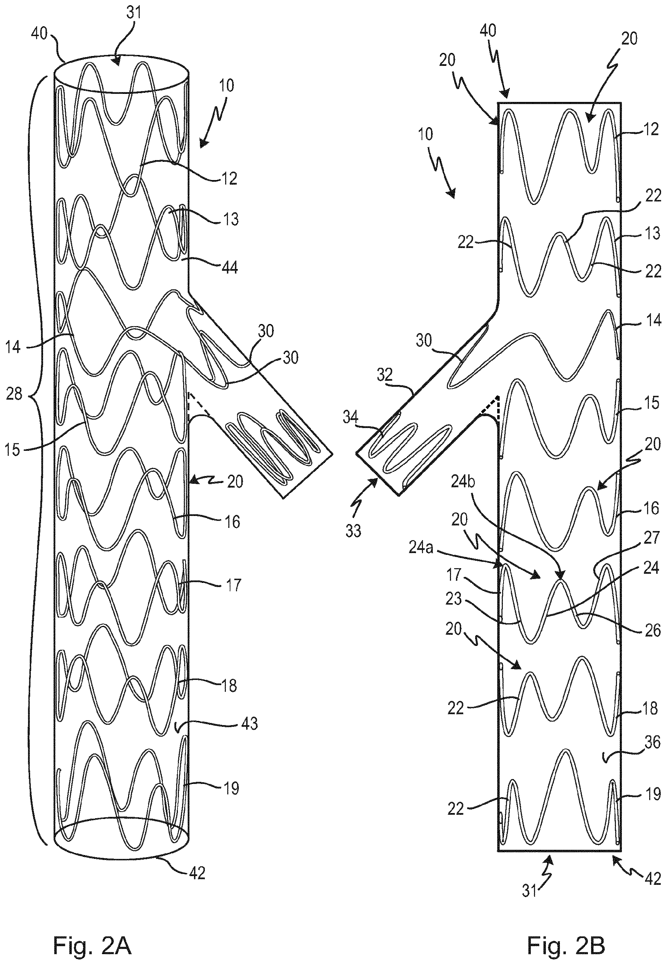

Fig. 2 zeigt insgesamt ein Ausführungsbeispiel eines erfindungsgemäßen Gefäßimplantats 10 mit hier beispielhaften acht hintereinander in Längsrichtung des Gefäßimplantats 10 angeordneten Stentfedern 12, 13, 14, 15, 16, 17, 18 und 19.

Fig. 2 ist ferner zu entnehmen, dass die einzelnen Stentfedern 12 bis 19 in unterschiedlichen Phasen angeordnete Spitzbögen 20 aufweisen. In Fig. 2 sind beispielhaft die Elemente der Stentfeder 18 mit Bezugszeichen versehen, wobei es sich versteht, dass die Stentfedern 12, 13, 14, 15, 16, 17, und 19 analog aufgebaut sind, und die Elementebezeichnung mit Bezugszeichen bei diesen lediglich aus Übersichtszwecken weggelassen wurden. Wie in Fig. 2a anhand der Stentfeder 18 gezeigt, ist ein Spitzbogen 20 jeweils aus zwei Schenkeln 22, 23, 26, 27 und entweder einem Scheitelpunkt 24a oder einem Tiefstpunkt 25a gebildet. Ein zum proximalen Ende 40 des Grundkörpers 44 weisender Spitzbogen 20 besitzt definitionsgemäß zumindest einen Scheitelpunkt 24a, ein zum distalen Ende weisender Spitzbogen 20 besitzt definitionsgemäß einen Tiefstpunkt 25a.

Das Gefäßimplantat 10 weist darüber hinaus insgesamt ein proximales Ende 40 und ein distales Ende 42 auf. Durch ein Implantatmaterial 43 sind die Stentfedern 12, 13, 14, 15, 16, 17, 18 und 19 indirekt - d.h. nicht direkt über Stege oder ähnliches - miteinander verbunden, so dass das Gefäßimplantat einen insgesamt hohlzylindrischen Grundkörper 44 mit einem Grundkörperlumen 31 aufweist, dessen Durchmesser sich über die Länge verändern kann oder nicht. Das in Fig. 2 gezeigte Gefäßimplantat weist daher einen sogenannten Abschnitt 28 auf, der "gestentet" ist, bzw. sie besteht aus einem solchen Abschnitt, da sämtliches Implantatmaterial 43 durch Stentfedern 12 bis 19, die in bestimmten Abständen zueinander an das Implantatmaterial 43 angebracht sind, unterstützt ist. Es versteht sich, dass das Gefäßimplantat 10 noch weitere Abschnitte aufweisen kann, die entweder aus einem mit einem Stent-Drahtgeflecht oder einem lasergeschnittenen Stent unterstützten Implantatmaterial bestehen, oder aus einem ungestenteten Implantatmaterial, oder aus einem Stent-Drahtgeflecht ohne Implantatmaterial.

Wie weiter oben erwähnt, weisen die Stentfedern 12, 13, 14, 15, 16, 17, 18 und 19 des in Fig. 2A und 2B gezeigten Ausführungsbeispiels des erfindungsgemäßen Gefäßimplantats 10 Spitzbögen 20 auf, mit Scheitelpunkten 24a und Tiefstpunkten 25a sowie mit diese verbindenden Schenkeln 23, 24, 26, 27. Fig. 2A und 2B ist ferner zu entnehmen, dass jeweils drei in umlaufender Richtung der Stentfeder 18 aufeinander folgende Schenkel 22, 23, 24, die einen Tiefpunkt 25b mit einem Scheitelpunkt 24a (Schenkel 22), den Scheitelpunkt 24a mit dem Tiefstpunkt 25a (Schenkel 23) und den Tiefstpunkt 25a mit dem Scheitelpunkt 24c (Schenkel 24) verbinden, unterschiedliche Längen besitzen: Die Länge des Schenkels 22 ist dabei kürzer als die Länge des Schenkels 23, und die Länge des Schenkels 24 ist wiederum länger als die Länge des Schenkels 23. Durch die abwechselnd unterschiedlichen Längen der Schenkel 22, 23, 24, 26, 27 ist der mäanderförmige Verlauf der Stentfedern 12, 13, 14, 15, 16, 17, 18 und 19, bzw. deren Scheitelpunkte 24a, 24b, 24c und Tiefstpunkte 25a, 25b, 25c, nicht gleichmäßig: Die Spitzbögen 20 einer Stentfeder 12, 13, 14, 15, 16, 17, 18 und 19 besitzen nicht die gleiche Höhe - basierend von einer gedachten Linie in Umfangsrichtung, die durch die Scheitelpunkte geführt ist -, sondern bilden aufgrund der unterschiedlich hohen Scheitel- bzw. Tiefstpunkte 24a, 24b, 24c, 25a, 25b, 25c unterschiedliche Phasen. Die Stentfedern 12, 13, 14, 15, 16, 17, 18 und 19 sind dadurch sozusagen asymmetrisch.

Anders gesagt, sind die Spitzbögen 20, die ihrer Form wegen vorliegend auch mit "Wellenberge"/"Wellentäler" bezeichnet werden, aufgrund der unterschiedlich langen Schenkel 22, 23, 24 unterschiedlich hoch bzw. tief. Bei dem asymmetrischen mäanderförmigen Verlauf der umlaufenden Stentfedern 12, 13, 14, 15, 16, 17, 18, 19 besitzen also jeweils zwei aufeinanderfolgende, in die proximale Richtung weisende Spitzbögen unterschiedliche Höhen, gemessen von einer gedachten geraden Linie, die die tiefsten Tiefpunkte 25b und 25c miteinander verbindet, und damit auch die jeweils dazwischen liegenden in die distale Richtung weisenden Spitzbögen unterschiedliche Tiefen.

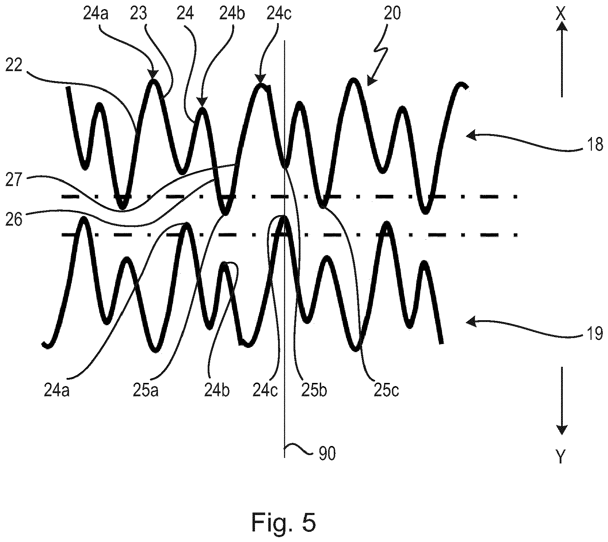

Darüber hinaus sind die Stentfedern 12, 13, 14, 15, 16, 17, 18 und 19 bei dem in Fig. 2A und 2B gezeigten Ausführungsbeispiel derart in Längsrichtung hintereinander angeordnet, derart, dass sich eine zusätzliche Asymmetrie ausbildet, was nachstehend auch in Fig. 5 näher erläutert wird.

Fig. 5 zeigt eine schematische, ausschnittsweise Darstellung zweier erfindungsgemäßer asymmetrischen Stentfedern 18, 19. Hier ist noch einmal vereinfachter das Prinzip der asymmetrischen Stentfedern gezeigt, wie es gemäß einer Ausführungsform des erfindungsgemäßen Gefäßimplantats verwirklicht werden kann: Die Stentfedern 18, 19 weisen jeweils Spitzbögen 20 auf, die abwechselnd in proximale und in distale Richtung weisen, und bilden dadurch Wellenberge und Wellentäler. Der Schenkel 22, Scheitelpunkt 24a, sowie der Schenkel 23 bilden dabei einen in proximale Richtung X weisenden Spitzbogen, und der Schenkel 23, der Tiefstpunkt 25a und der Schenkel 24 bilden einen in distale Richtung Y weisenden Spitzbogen. Aufgrund der unterschiedlichen Längen der aufeinander folgenden Schenkel 22, 23, 24, 26, 27, weisen die Spitzbögen unterschiedliche Höhen bzw. Tiefen auf, was an den unterschiedlich hohen Scheitelpunkten 24a, 24b, bzw. den unterschiedlich tiefen Tiefstpunkten 25a, 25b zu erkennen ist, deren Höhe bzw. Tiefe anhand einer gedachten umlaufenden Linie senkrecht zur Längsrichtung des Gefäßimplantats bemessen wird. So folgt also in umlaufender Richtung abwechselnd auf einen höheren Scheitelpunkt 24a ein Scheitelpunkt 24b, der niedriger bzw. weniger hoch ist als der vorherige Scheitelpunkt 24a, und auf diesen Scheitelpunkt 24b wieder ein Scheitelpunkt 24c, der höher ist als der Scheitelpunkt 24b unmittelbar davor, usw., wobei zwischen den unterschiedlich hohen Scheitelpunkten 24a, 24b, 24c jeweils unterschiedlich tiefe Tiefstpunkte 25a, 25b, 25c liegen: Auch bei den Tiefstpunkten 25a, 25b, 25c folgt in umlaufender Richtung abwechselnd auf einen tieferen Tiefstpunkt 25a wieder ein Tiefstpunkt 25b, der weniger tief ist als der in umlaufender Richtung unmittelbar vorher angeordnete Tiefstpunkt 25a, und auf diesen Tiefstpunkt 25b wieder ein Tiefstpunkt 25c, der tiefer ist als der davor. Anders gesprochen besitzt eine Stentfeder 18, 19 also Spitzbögen 20 mit mindestens zwei oder drei unterschiedlich tiefen Tiefstpunkten 25a, 25b, 25c, wobei sich die unterschiedliche Tiefen auf eine senkrecht zur Längsrichtung des hohlzylindrischen Grundkörpers umlaufende, gedachte Linie beziehen, welche die tiefsten Tiefstpunkte 25a, 25c miteinander verbindet. Definitionsgemäß liegen damit weniger tiefe Tiefstpunkte 25b nicht auf dieser gedachten Linie, und gleiches gilt wiederum für die Scheitelpunkte.

Wie Fig. 5 ferner zu entnehmen ist, sind die zwei in Längsrichtung hintereinander angeordnete Stentfedern 18, 19 in Bezug auf die Längsrichtung des hohlzylindrischen Grundkörpers und in Bezug aufeinander derart angeordnet sind, dass die Tiefstpunkte 25a, 25b, 25c der Spitzbögen 20 einer ersten Stentfeder 18 jeweils den Scheitelpunkten 24a, 24b, 24c von Spitzbögen 20 einer in Längsrichtung des Gefäßimplantats 10 der ersten Stentfeder 18 nachgeordneten zweiten Stentfeder 19 in einem Abstand und in einer gedachten Linie 90, die parallel zur Längsrichtung des Gefäßimplantats 10 ist, gegenüber liegen, und zwar derart, dass ein tiefster Tiefstpunkt 25a eines Spitzbogens 20 der ersten Stentfeder 18 einem Scheitelpunkt 24b der distal nachgeordneten zweiten Stentfeder 19 in einem Abstand gegenüber liegt, wobei dieser Scheitelpunkt 24b weniger hoch ist als ein höchster Scheitelpunkt 24a der zweiten Stentfeder 19; entsprechend kommt damit bei dieser Ausführungsform immer auch ein weniger tiefer Tiefstpunkt 25b - also ein Tiefstpunkt 25b, der weniger tief ist als ein tiefster Tiefpunkt 25a der ersten Stentfeder 18- in einem Abstand zu und gegenüber einem höchsten Scheitelpunkt 24c der distal nachgeordneten zweiten Stentfeder 19 zu liegen, welcher höchste Scheitelpunkt 24c höher ist als ein weniger hoher Scheitelpunkt 24b der zweiten Stentfeder 19.

Anders gesprochen zeigt Fig. 5 ein Beispiel für die Ausführung von Stentfedern des erfindungsgemäßen Gefäßimplantats, bei welcher Ausführung zumindest zwei in Längsrichtung hintereinander angeordnete Stentfedern 18, 19 jeweils Schenkel 22, 23, 24 mit unterschiedlichen Längen aufweisen zur Ausbildung von umlaufend aufeinander folgenden unterschiedlich hohen Spitzbögen 24a, 24b, 24c; 25a, 25b, 25c, mit höheren 24a, 24c; 25a, 25c und kürzeren 24b; 25b Spitzbögen, und dass ein in proximale Richtung x weisender höherer Spitzbogen 24a einer ersten Stentfeder 19 einem in distale Richtung y weisenden kürzeren Spitzbogen 25b einer in proximale Richtung X nachgeordneten zweiten Stentfeder 18 in einem Abstand und in einer gedachten Linie 90 gegenüber liegt, die parallel zur Längsrichtung des Gefäßimplantats 10 ist, und/oder dass ein in proximale Richtung x weisender kürzerer Spitzbogen (24b) einer ersten Stentfeder (19) einem in distale Richtung y weisenden längeren Spitzbogen (25a, 25c) einer in proximale Richtung x nachgeordneten zweiten Stentfeder (18) in einem Abstand und in einer gedachten Linie 90 gegenüber liegt, die parallel zur Längsrichtung des Gefäßimplantats 10 ist.

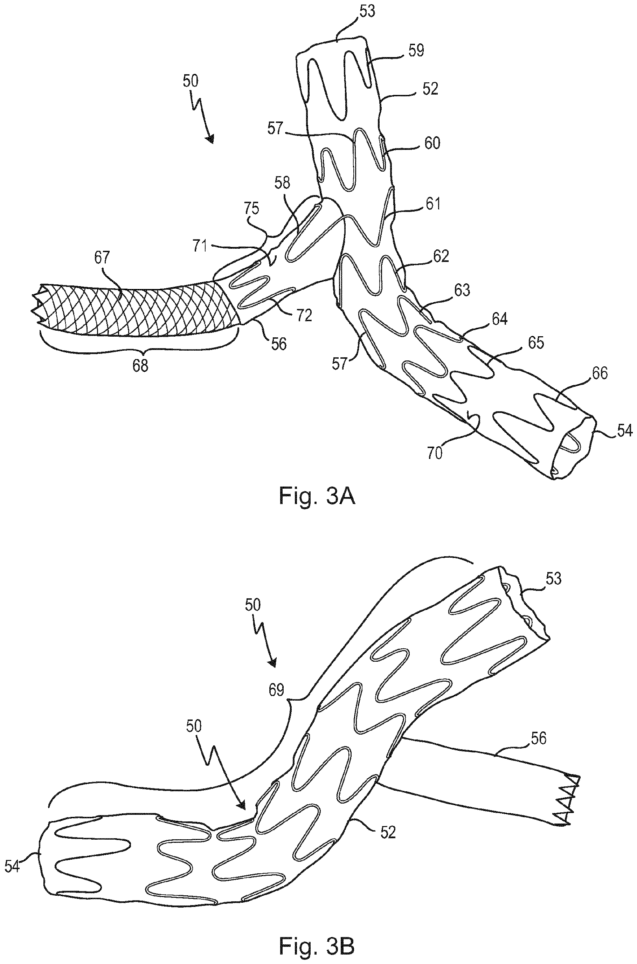

Zurück zu Fig. 2A, die die Ausführungsform eines erfindungsgemäßen Gefäßimplantats in räumlicher Darstellung zeigt (das die Stentfedern verbindende Implantatmaterial ist in Fig. 2A der Übersicht halber nicht dargestellt): Fig. 2A ist ferner zu entnehmen, dass die Stentfeder zwei ausgestellte Spitzbögen 30 aufweist, die aufgrund ihrer Ausformung bezüglich der Längsrichtung des Gefäßimplantats 10 seitlich in einem Winkel abstehen. Die übrigen Spitzbögen 20 aller Stentfedern 12, 13, 14, 15, 16, 17, 18 und 19 verlaufen parallel zur Längsrichtung des Gefäßimplantats 10. Durch dieses Abstehen bzw. durch die Aufstellung der Spitzbögen 30 der Stentfeder 14 wird ein Seitenkörper bzw. Seitenast 32 aufgespannt; dieser Seitenast 32 bietet im Seitengefäß eine Verankerungszone für einen bspw. gecoverten Stentgraft/Stentgraft-Abschnitt, der in ein von einem Hauptblutgefäß abzweigendes Seitengefäß eingeführt wird (siehe Fig. 3A).

In dem in Fig. 2A und 2B gezeigten Beispiel weist der Seitenkörper 32 noch eine eigene Seitenkörper-Stentfeder 34 auf, die damit keine Stentfeder des Grundkörpers 44 des Gefäßimplantats 10 bildet. Die Seitenkörper-Stentfeder 34 ist indirekt durch ein Seitenkörper-Implantatmaterial 36 mit dem ausgestellten Spitzbogen 30 der Stentfeder 14 des Grundköpers 44 verbunden.

In dem in Fig. 2A und 2B gezeigten Beispiel sind die Stentfedern 12 bis 19 und die Seitenkörper-Stentfeder 34 an das Implantatmaterial 43 bzw. 36 aufgenäht.

Fig. 2A und 2B ist ferner zu entnehmen, dass die Spitzbögen 20 der einzelnen Stentfedern 12 bis 19 phasenversetzt in Bezug auf die jeweils nachgeordnete Stentfeder 12 bis 19 angeordnet sind, so dass einem Scheitelpunkt 24 einer ersten Stentfeder 12 jeweils in einer senkrecht in Längsrichtung gedachten Linie ein weiterer Scheitelpunkt 24 bzw. jeweils ein Tiefstpunkt 25 der nachgeordneten Stentfeder 13 folgt, sondern diese schräg gegeneinander versetzt sind.

Fig. 3 zeigt eine weitere Ausführungsform eines erfindungsgemäßen Gefäßimplantats 50, ebenfalls mit einem Grundkörper 52, einem proximalen Ende 53 und einem distalen Ende 54.

Das in Fig. 3A und B in unterschiedlichen Beugungspositionen dargestellte Gefäßimplantat 50 weist ferner einen Seitenkörper 56 auf, der durch zwei ausgestellte Spitzbögen 58 einer Stentfeder 61 gebildet wird (nur ein ausgestellter sichtbar).

Der Grundkörper 52 des Gefäßimplantats 50 weist insgesamt acht Stentfedern 59, 60, 61, 62, 63, 64, 65, 66 mit mäanderförmig umlaufenden Spitzbögen 57 auf und weist daher insgesamt einen Abschnitt 69 auf, der aufgrund der unterstützenden Stentfedern 59 bis 66 als gestentet betrachtet/bezeichnet wird. Der Seitenkörper bzw. -ast 56 weist eine eigene Seitenkörper-Stentfeder 72 auf, sowie einen durch ein Stent-Drahtgeflecht 67 unterstützten Abschnitt 68. Sowohl der Grundköper 52 des Gefäßimplantats 50 als auch deren Seitenkörper 56 besitzen jeweils ein Implantatmaterial, wobei in Fig. 3 das Implantatmaterial des Grundkörpers 52 mit 70 und das Implantatmaterial des Seitenkörpers 56 mit 71 bezeichnet ist.

Fig. 3A und B ist ferner zu entnehmen, dass der Seitenkörper 56 neben einem gestenteten Seitenkörper-Abschnitt 75 den weiteren Abschnitt 68 aufweist, bei welchem das Implantatmaterial 71 vom Stent-Drahtgeflecht 67 unterstützt ist. Das Seitenkörper-Implantatmaterial 71 kann in diesem Abschnitt 68 unterschiedlich zu dem Seitenkörper-Implantatmaterial 71 im gestenteten Abschnitt 75 des Seitenkörpers 56 sein, und bspw. dichter, fester oder aus einem anderen Material gefertigt sein.

Dieser Seitenkörper-Abschnitt 75 aus Stent-Drahtgeflecht 67 und Implantatmaterial 71 stellt einen gecoverten Stentgraft dar, der vorzugsweise erst *in vivo,* also nachdem der Grundkörper 52 bereits in das Hauptgefäß implantiert wurde, in diese Position implantiert wird.

Ähnlich kann gemäß einer anderen Ausführungsform der Grundkörper 44 oder 52 des Gefäßimplantats 10, 50 einen weiteren Abschnitt aufweisen, der durch ein Stent-Drahtgeflecht unterstützt ist, oder aber insgesamt ungestentet ist, und lediglich aus Implantatmaterial gebildet ist, oder aber nur ein Stent-Drahtgeflecht aufweist.

Auch ist Fig. 3A und B bzw. dem in Fig. 3A und B gezeigten erfindungsgemäßen Gefäßimplantat 50 zu entnehmen, dass durch die ausgestellten Spitzbögen (nur einer sichtbar) 58 der Stentfeder 61 des Grundkörpers 52 der Seitenkörper 56 des Gefäßimplantats 50 insgesamt von der Längsrichtung des Grundkörpers 52 in einem Winkel absteht.

Wie bereits weiter oben erwähnt, ist der Winkel, mit dem der Seitenkörper 56 vom Grundkörper 52 absteht, auch hier flexibel und frei wählbar, und kann in Abhängigkeit von dem abzweigenden Blutgefäß bzw. dessen Winkel ausgewählt werden. Bevorzugt sind Winkel im Bereich zwischen 35° und 90°, und insbesondere Winkel im Bereich 40° bis 50°, und noch bevorzugter im Bereich zwischen 44° bis 46°.

In Fig. 3A und 3B ist die dargestellte Ausführungsform des erfindungsgemäßen Gefäßimplantats 50 in einem gebogenen Zustand des Grundkörpers 52 gezeigt, wobei hier ersichtlich ist, dass aufgrund der asymmetrischen Ausbildung der Phasen und aufgrund der gegeneinander versetzten Stentfedern 59, 60, 61, 62, 63, 64, 65, 66 eine Knickbildung im Bereich einer Implantatbeugung 74 vermieden wird. Der erreichbare Biegeradius (Flexibilität) ist deutlich kleiner als bei symmetrischen Stentfedern bei gleichbleibender Längsstabilität.

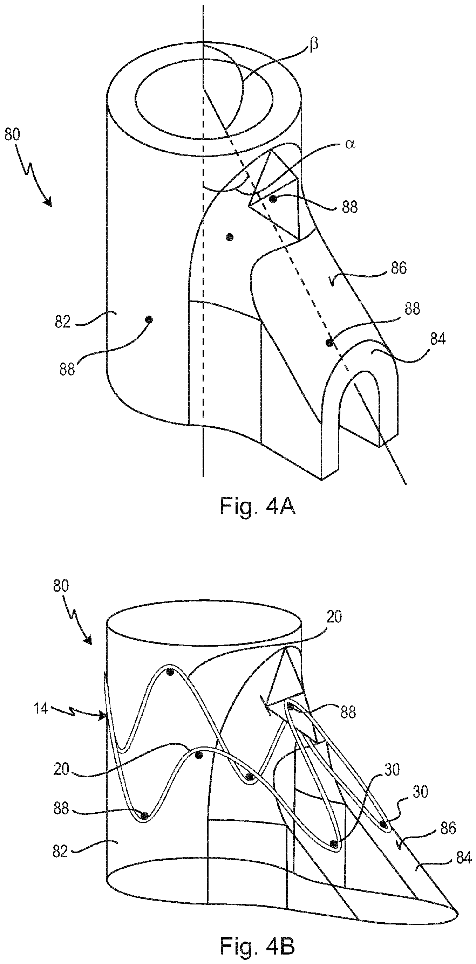

Fig. 4A schließlich zeigt in a mit dem Bezugszeichen 80 insgesamt ein Formstück, das einen zylinderförmigen Grundkörper 82 sowie einen winklig vom zylindrischen Grundkörper 82 abstehendes und mit dem zylinderförmigen Grundkörper 82 verbundenes Formstückelement 84 aufweist. Das Formstückelement 84 steht dabei im Verhältnis zur Längsachse des zylinderförmigen Grundkörpers 82 mit einem Winkel α ab und bildet eine abgerundete schräge Oberfläche 86, deren Neigung bezüglich des zylindrischen Grundkörper 82 mit dem Winkel β bezeichnet ist. Das Formstück weist ferner stiftartige oder stiftförmige Elemente 88 auf, die zur Ausbildung der mäanderförmig umlaufenden Wellen oder Spitzbögen 20, 30 bspw. der Stentfeder 14 vorgesehen sind (siehe Fig. 4B).

Zur Ausbildung eines erfindungsgemäßen Gefäßimplantats 10, 50 wird dieses Formstück 80 derart eingesetzt, dass hierauf diejenige Stentfeder ausgeformt wird, die zumindest einen, vorzugsweise zumindest zwei, ausgestellten Spitzbogen (Spitzbögen) 30 bzw. 58 aufweist (siehe Fig. 4B). In Fig. 4B ist zur besseren Übersichtlichkeit das Formstück transparent dargestellt. Zur Bildung einer Stentfeder 14 wird bspw. ein Metalldraht mit Shape-Memory-Eigenschaft, bspw. aus Nitinol, im kalten Zustand um die stiftförmigen Elemente 88 des zylinderförmigen Grundkörpers 82 umlaufend in mäanderförmigen Schleifen geführt, um die Spitzbögen 20 zu bilden. Der ausgestellte Spitzbogen 30 wird dadurch gebildet, dass dieser über die schräge Oberfläche 86 des Formstückelements 84 gelegt und auch dort über die stiftförmigen Elemente 88 geführt wird. Der Draht wird fixiert und anschließend einer Wärmebehandlung unterzogen, um die Mäander-Form zu fixieren.

Nach Aushärten des erwärmten Materials behält die so gebildete Stentfeder ihre Form, die den expandierten Zustand der Stentfeder darstellt.

Die so gebildete Stentfeder wird an der gewünschten Stelle in dem Gefäßimplantat 10, 50 platziert, mithin also an derjenigen Stelle des Gefäßimplantats 10, 50, von der aus der zumindest eine Seitenkörper 32, 56 des Gefäßimplantats 10, 50 aufgestellt werden soll.

## Patentansprüche

1. Gefäßimplantat (10; 50) zur Implantation in ein Blutgefäß eines Patienten, wobei das Gefäßimplantat (10; 50) von einem komprimierten Zustand in einen selbstexpandierten Zustand überführbar ist, mit einem hohlzylindrischen Grundkörper (44; 52) mit einem proximalen (40; 53) und einem distalen Ende (42; 54), einem Grundkörperlumen (31) und einer Längsrichtung, und mit einem Abschnitt (28; 69), der in der Längsrichtung des Grundkörpers (44; 52) hintereinander angeordnete und senkrecht zur Längsrichtung jeweils mäanderförmig umlaufende jeweils einstückige Stentfedern (12, 13, 14, 15, 16, 17, 18, 19; 59, 60, 61, 62, 63, 46, 65, 66) und ein an den Stentfedern (12, 13, 14, 15, 16, 17, 18, 19; 59, 60, 61, 62, 63, 46, 65, 66) befestigtes und diese verbindendes Implantatmaterial (43; 70) aufweist, wobei die Stentfedern (12, 13, 14, 15, 16, 17, 18, 19; 59, 60, 61, 62, 63, 46, 65, 66) lediglich über das Implantatmaterial (43; 70) und nicht untereinander verbunden sind, und wobei die mäanderförmig umlaufenden Stentfedern (12, 13, 14, 15, 16, 17, 18, 19; 59, 60, 61, 62, 63, 46, 65, 66) abwechselnd zum proximalen und distalen Ende des Grundkörpers (44; 52) und parallel zu dessen Längsrichtung weisende Spitzbögen (20; 57) aufweisen, und ferner mit zumindest einem vom Grundkörper (44; 52) abzweigenden hohlzylindrischen Seitenkörper (32; 56) mit einem Seitenkörperlumen und einem Seitenkörper-Implantatmaterial (36; 71), wobei das Seitenkörperlumen (33) mit dem Grundkörperlumen (31) in Fluidverbindung steht, **dadurch gekennzeichnet, dass** der zumindest eine vom Grundkörper (44; 52) abzweigende Seitenkörper (32; 56) im selbstexpandierten Zustand in einem Winkel bezüglich der Längsrichtung des Grundkörpers (44; 52) ausgestellt ist, wobei der Winkel durch eine im Verhältnis zur Längsrichtung des Grundkörpers (44, 52) vorgeformte abgewinkelte Ausstellung zumindest eines Spitzbogens (30; 58) einer Stentfeder (14; 61) des Grundkörpers (44, 52) im selbstexpandierten Zustand bewirkt ist.

2. Gefäßimplantat (10; 50) nach Anspruch 1, **dadurch gekennzeichnet, dass** ein Spitzbogen (20; 30; 57; 58) jeweils aus zwei Schenkeln (23, 24; 26; 27) und einem zwischen den Schenkeln (23, 24; 26, 27) liegenden Scheitelpunkt (24a, 24b, 24c) bzw. Tiefstpunkt (25a, 25b, 25c) gebildet ist.

3. Gefäßimplantat (10; 50) nach Anspruch 1 oder 2, **dadurch gekennzeichnet, dass** der Winkel zwischen 20° und 90° beträgt, und insbesondere zwischen 35° und 55°, und vorzugsweise 45° beträgt.

4. Gefäßimplantat (10; 50) nach einem der vorstehenden Ansprüche, **dadurch gekennzeichnet, dass** der zumindest eine Seitenkörper (32; 56) einen ersten Seitenkörper-Abschnitt (75) aufweist mit zumindest einer mäanderförmig umlaufenden Seitenkörper-Stentfeder (34; 72) und einem an der Seitenkörper-Stentfeder (34; 72) und der abgewinkelt ausgestellten Stentfeder (14; 61) des Grundkörpers (44; 52) befestigten und diese verbindenden Seitenkörper-Implantatmaterial (36; 71), wobei die zumindest eine Seitenkörper-Stentfeder (34; 72) und die abgewinkelt ausgestellte Stentfeder (14; 61) des Grundkörpers (44; 52) lediglich über das Seitenkörper-Implantatmaterial (36; 71) und nicht untereinander verbunden sind.

5. Gefäßimplantat (10; 50) nach einem der vorstehenden Ansprüche, **dadurch gekennzeichnet, dass** der Grundkörper (44; 52) und/oder der zumindest eine Seitenkörper (32; 56) ferner zusätzlich zumindest einen weiteren Abschnitt (76) aufweist, der keine Stentfedern (12, 13, 14, 15, 16, 17, 18, 19; 59, 60, 61, 62, 63, 46, 65, 66) aufweist, oder der einen geflochtenen oder gelaserten Stent (77) aufweist, wobei sich der zumindest eine weitere Abschnitt (76) proximal oder distal an den Abschnitt (28; 69; 75) anschließt.

6. Gefäßimplantat (10; 50) nach einem der vorstehenden Ansprüche 2 bis 5, **dadurch gekennzeichnet, dass** zumindest drei Schenkel (22, 23, 26), die einen ersten Scheitelpunkt (24a), einen auf den ersten Scheitelpunkt (24a) in umlaufenden Richtung folgenden ersten Tiefstpunkt (25a), einen dem ersten Tiefstpunkt (25a) in umlaufender Richtung folgenden zweiten Scheitelpunkt (24b), und einem dem zweiten Scheitelpunkt (24b) in umlaufender Richtung folgenden zweiten Tiefstpunkt (25b) verbinden, jeweils unterschiedliche Längen aufweisen, wodurch ein mäanderförmiger Verlauf einer Stentfeder (12, 13, 14, 15, 16, 17, 18, 19; 59, 60, 61, 62, 63, 46, 65, 66) mit unregelmäßigen Spitzbögen (20; 57) gebildet wird.

7. Gefäßimplantat (10; 50) nach einem der Ansprüche 1 bis 6, **dadurch gekennzeichnet, dass** zumindest zwei in Längsrichtung hintereinander angeordnete Stentfedern (12, 13, 14, 15, 16, 17, 18, 19; 59, 60, 61, 62, 63, 46, 65, 66) in Bezug auf die Längsrichtung des hohlzylindrischen Grundkörpers (44; 52) und mit Bezug aufeinander derart angeordnet sind, dass die Scheitelpunkte (24a, 24b, 24c) der Spitzbögen (20; 57) einer ersten Stentfeder (12, 13, 14, 15, 16, 17, 18, 19; 59, 60, 61, 62, 63, 46, 65, 66) jeweils Tiefstpunkten (25a, 25b, 25c) von Spitzbögen (20; 57) einer in Längsrichtung des Gefäßimplantats (10;50) nachgeordenten zweiten Stentfeder (12, 13, 14, 15, 16, 17, 18, 19; 59, 60, 61, 62, 63, 46, 65, 66) in einem Abstand voneinander und in einer Linie, die parallel zur Längsrichtung des Gefäßimplantats ist, gegenüber liegen.

8. Gefäßimplantat (10; 50) nach einem der Ansprüche 2 bis 5, **dadurch gekennzeichnet, dass** zumindest zwei in Längsrichtung hintereinander angeordnete Stentfedern (12, 13, 14, 15, 16, 17, 18, 19; 59, 60, 61, 62, 63, 46, 65, 66) jeweils Schenkel (23, 24; 26, 27) mit unterschiedlichen Längen aufweisen zur Ausbildung von umlaufend aufeinander folgenden unterschiedlich hohen Spitzbögen (20; 30; 57; 58), mit höheren (24a, 24c; 25a, 25c) und kürzeren (24b; 25b) Spitzbögen, und dass ein in proximale Richtung x weisender höherer Spitzbogen (24a, 24c) einer ersten Stentfeder (19) einem in distale Richtung y weisenden kürzeren Spitzbogen (25b) einer proximal nachgeordneten zweiten Stentfeder (18) in einem Abstand und in einer gedachten Linie gegenüber liegt, die parallel zur Längsrichtung des Gefäßimplantats (10) ist, und/oder dass ein in proximale Richtung x weisender kürzerer Spitzbogen (24b) einer ersten Stentfeder (19) einem in distale Richtung y weisenden längeren Spitzbogen (25a) einer in proximale Richtung x nachgeordneten zweiten Stentfeder (18) in einem Abstand und in einer gedachten Linie (90) gegenüber liegt, die parallel zur Längsrichtung des Gefäßimplantats (10; 50) ist.

9. Gefäßimplantat (10; 50) nach einem der Ansprüche 1 bis 8, **dadurch gekennzeichnet, dass** das Seitenkörper-Implantatmaterial (36; 71) mit dem Implantatmaterial (43; 70) des Grundkörpers (44; 52) verbunden, vorzugsweise vernäht, ist.

10. Gefäßimplantat (10; 50) nach einem der Ansprüche 1 bis 9, **dadurch gekennzeichnet, dass** die Stentfedern (12, 13, 14, 15, 16, 17, 18, 19; 59, 60, 61, 62, 63, 46, 65, 66) des Grundkörpers (44; 52) und/oder des Seitenkörpers (32; 56) innen an oder außen auf das Implantatmaterial (43; 70; 36; 71) des hohlzylindrischen Grundkörpers (44; 52) und/oder des Seitenkörpers (32; 56) angebracht sind.

11. Gefäßimplantat (10; 50) nach einem der Ansprüche 1 bis 10, **dadurch gekennzeichnet, dass** zwischen einem und vier vom Grundkörper (44; 52) winklig ausgestellte Seitenkörper (32; 56) vorgesehen sind.

12. Verfahren zur Herstellung eines Gefäßimplantats (10; 50) nach einem der Ansprüche 1 bis 11, **dadurch gekennzeichnet, dass** es die folgenden Schritte aufweist:
- Bereitstellen eines Formstücks (80), mit einem zylinderförmigen Grundkörper (82) und mit einem winklig vom zylinderförmigen Grundkörper (82) abstehendes und mit dem Formstück (80) fest verbundenes Formstückelement (84) aufweist, welches eine abgerundete schräge Fläche (86) aufweist, und wobei das Formstück (80) ferner stiftförmige Elemente (88) in der Position von Scheitel- und Tiefstpunkten mäanderförmig umlaufender Stentfedern aufweist,
- Bereitstellen eines Stentfeder-bildenden Drahtes, vorzugsweise aus Nitinol,
- Aufwickeln des Stentfeder-bildenden Drahtes unter Zug auf dem Formstück (80) und über die stiftförmigen Elemente 88), derart, dass mäanderförmig umlaufenden Stentfedern (12, 13, 14, 15, 16, 17, 18, 19; 59, 60, 61, 62, 63, 46, 65, 66) mit Spitzbögen (20; 30) um das Formstück (80) gewickelt und auf dem Formstück (80) fixiert werden, wobei auf dem winklig abstehenden Formstückelement (84) der zumindest eine winklig ausgestellte Spitzbogen (30; 58) ausgebildet wird.

## Claims

1. A vascular implant (10; 50) for implanting into a blood vessel of a patient, wherein the vascular implant (10; 50) is being transformable from a compressed state into a self-expanded state, comprising a hollow-cylindrical main body (44; 52) with a proximal end (40; 53) and a distal end (42; 54), a main body lumen (31) and a longitudinal direction, and comprising a portion (28; 69) that comprises, respectively, one-piece stent springs (12, 13, 14, 15, 16, 17, 18, 19; 59, 60, 61, 62, 63, 46, 65, 66) successively arranged in the longitudinal direction of the main body (44; 52) and circumferentially meandering, respectively, perpendicularly in relation to the longitudinal direction, and an implant material (43; 70) fixed to the stent springs (12, 13, 14, 15, 16, 17, 18, 19; 59, 60, 61, 62, 63, 46, 65, 66) and connecting them, the stent springs (12, 13, 14, 15, 16, 17, 18, 19; 59, 60, 61, 62, 63, 46, 65, 66) only being connected to one another via the implant material (43; 70) and not between one another, and wherein the circumferentially meandering stent springs (12, 13, 14, 15, 16, 17, 18, 19; 59, 60, 61, 62, 63, 46, 65, 66) comprise pointed arches (20; 57) that alternately face toward the proximal end and the distal end of the main body (44; 52) and parallel to the longitudinal direction thereof, and also comprising at least one hollow-cylindrical side-body (32; 56) that branches off from the main body (44; 52) and comprises a side-body lumen and a side-body implant material (36; 71), wherein the side-body lumen (33) being a fluidic connection with the main body lumen (31), **characterized in that**, in the self-expanded state, the at least one side-body (32; 56) branching off from the main body (44; 52) is set up at an angle in relation to the longitudinal direction of the main body (44; 52), wherein the angle is being effected by at least one pointed arch (30; 48) of a stent spring (14; 61) of the main body (44, 52) angularly protruding in a performed way in relation to the longitudinal direction of the main body (44, 52) in the self-expanded state.

2. The vascular implant (10; 50) as claimed in claim 1, **characterized in that** a pointed arch (20; 30; 57; 58) is respectively formed by two legs (23, 24; 26; 27) and a vertex (24a, 24b, 24c) or lowest point (25a, 25b, 25c) lying between the legs (23, 24; 26, 27).

3. The vascular implant (10; 50) as claimed in claim 1 or 2, **characterized in that** the angle is between 20° and 90°, and in particular between 35° and 55°, and is preferably 45°.

4. The vascular implant (10; 50) as claimed in one of the preceding claims, **characterized in that** the at least one side-body (32; 56) comprises a first side-body portion (75) with at least one circumferentially meandering side-body stent spring (34; 72) and a side-body implant material (36; 71) fixed to the side-body stent spring (34; 72) and the angularly protruding stent spring (14; 61) of the main body (44; 52) and connecting them, wherein the at least one side-body stent spring (34; 72) and the angularly protruding stent spring (14; 61) of the main body (44; 52) only being connected via the side-body implant material (36; 71) and not between one another.

5. The vascular implant (10; 50) as claimed in one of the preceding claims, **characterized in that** the main body (44; 52) and/or the at least one side-body (32; 56) additionally comprises at least one further portion (76) that does not comprise any stent springs (12, 13, 14, 15, 16, 17, 18, 19; 59, 60, 61, 62, 63, 46, 65, 66) or that comprises a braided or lasered stent (77), wherein the at least one further portion (76) adjoining the portion (28; 69; 75) proximally or distally.

6. The vascular implant (10; 50) as claimed in one of the preceding claims 2 to 5, **characterized in that** at least three legs (22, 23, 26), which connect a first vertex (24a), a first lowest point (25a) following the first vertex (24a) in a circumferential direction, a second vertex (24b) following the first lowest point (25a) in the circumferential direction, and a second lowest point (25b) following the second vertex (24b) in the circumferential direction, comprise different legths, respectively, whereby a meandering shape of a stent spring (12, 13, 14, 15, 16, 17, 18, 19; 59, 60, 61, 62, 63, 46, 65, 66) with irregular pointed arch (20; 57) is formed.

7. The vascular implant (10; 50) as claimed in one of the claims 1 to 6, **characterized in that** at least two stent springs (12, 13, 14, 15, 16, 17, 18, 19; 59, 60, 61, 62, 63, 46, 65, 66) arranged successively in the longitudinal direction are arranged with respect to the longitudinal direction of the hollow-cylindrical main body (44; 52) and with respect to one and another, such, that the vertices (24a, 24b, 24c) of the pointed arches (20; 57) of the first stent springs (12, 13, 14, 15, 16, 17, 18, 19; 59, 60, 61, 62, 63, 46, 65, 66) respectively lie opposite lowest points (25a, 25b, 25c) of pointed arches (20; 57) of a second stent spring (12, 13, 14, 15, 16, 17, 18, 19; 59, 60, 61, 62, 63, 46, 65, 66) arranged thereafter in a longitudinal direction of the vascular implant (10; 50) at a distance from one and another in a line that is parallel to the longitudinal direction of the vascular implant.

8. The vascular implant (10; 50) as claimed in one of the claims 2 to 5, **characterized in that** at least two stent springs (12, 13, 14, 15, 16, 17, 18, 19; 59, 60, 61, 62, 63, 46, 65, 66) successively arranged in the longitudinal direction respectively comprise legs (23, 24; 26, 27) of different lengths for forming of the pointed arches (20; 30; 57; 58) of varying height circumferentially following one and another, with higher pointed arches (24a, 24c; 25a, 25c) and shorter pointed arches (24b; 25b), and **in that** a higher pointed arch (24a, 24c), facing towards the proximal direction x, of a first stent spring (19) lies opposite a shorter pointed arch (25b), facing towards the distal direction y, of a second stent spring (18) arranged proximally thereafter, at a distance and in an imaginary line that is parallel to the longitudinal direction of the vascular implant (10) and/or **in that** a shorter pointed arch (24b), facing towards the proximal direction x, of a first stent spring (19) lies opposite a longer pointed arch (25a), facing toward the distal direction y, of a second stent spring (18) arranged thereafter in the proximal direction x, at a distance and in an imaginary line (90) that is parallel to the longitudinal direction of the vascular implant (10; 50).

9. The vascular implant (10; 50) as claimed in one of the claims 1 to 8, **characterized in that** the side-body implant material (36; 71) is connected, preferably sewn, to the implant material (43; 70) of the main body (44; 52).

10. The vascular implant (10; 50) as claimed in one of the claims 1 to 9, **characterized in that** the stent springs (12, 13, 14, 15, 16, 17, 18, 19; 59, 60, 61, 62, 63, 46, 65, 66) of the main body (44; 52) and/or the side-body (32; 56) are attached on the inside or the outside to the implant material (43; 70; 36; 71) of the hollow-cylindrical main body (44; 52) and/or of the side-body (32; 56).

11. The vascular implant (10; 50) as claimed in one of the claims 1 to 10, **characterized in that** between one and four side-bodies (32; 56) set out at an angle from the main body (44; 52) are provided.

12. A method for producing a vascular implant (10; 50) as claimed in one of the claims 1 to 11, **characterized in that** it comprises the following steps:
- providing a fitting (80), with a cylindrical main body (82) and with a fitting element (84), which protrudes at an angle from the cylindrical main body (82) and is fixedly connected to the fitting (80) and which comprises a rounded inclined face (86), and wherein the fitting (80) also comprises pin-shaped elements (88) in the position of vertices and lowest points of circumferentially meandering stent springs,
- providing a stent-spring-forming wire, preferably of nitinol,
- winding the stent-spring-forming wire under tension on the fitting (80) and over the spin-shaped elements (88), in such a way that circumferentially meandering stent springs (12, 13, 14, 15, 16, 17, 18, 19; 59, 60, 61, 62, 63, 46, 65, 66) with pointed arches (20; 30) are wound around the fitting (80) and are fixed on the fitting (80), wherein the at least one pointed arch (30; 58) set out at an angle being formed on the fitting element (84) protruding at an angle.

## Revendications

1. Implant vasculaire (10 ; 50) destiné à être implanté dans un vaisseau sanguin d'un patient, l'implant vasculaire (10 ; 50) pouvant être amené d'un état comprimé à un état auto-expansé, comprenant un corps de base cylindrique creux (44 ; 52) avec une extrémité proximale (40 ; 53) et une extrémité distale (42 ; 54), une lumière de corps de base (31) et une direction longitudinale, et une portion (28 ; 69), qui présente des ressorts d'endoprothèse d'une seule pièce (12, 13, 14, 15, 16, 17, 18, 19 ; 59, 60, 61, 62, 63, 46, 65, 66) disposés les uns derrière les autres dans la direction longitudinale du corps de base (44 ; 52) et enroulés à chaque fois en méandres perpendiculairement à la direction longitudinale et un matériau d'implant (43 ; 70) fixé aux ressorts d'endoprothèse (12, 13, 14, 15, 16, 17, 18, 19 ; 59, 60, 61, 62, 63, 46, 65, 66) et reliant ceux-ci, les ressorts d'endoprothèse (12, 13, 14, 15, 16, 17, 18, 19 ; 59, 60, 61, 62, 63, 46, 65, 66) étant seulement connectés par le biais du matériau d'implant (43 ; 70) et n'étant pas connectés les uns aux autres, et les ressorts d'endoprothèse enroulés en forme de méandres (12, 13, 14, 15, 16, 17, 18, 19 ; 59, 60, 61, 62, 63, 46, 65, 66) présentant des portions courbées en pointe (20 ; 57) tournées en alternance vers l'extrémité proximale et vers l'extrémité distale du corps de base (44 ; 52) et parallèlement à sa direction longitudinale, et comprenant en outre au moins un corps latéral cylindrique creux (32 ; 56) partant du corps de base (44 ; 52) avec une lumière de corps latéral et un matériau d'implant de corps latéral (36 ; 71), la lumière de corps latéral (33) étant en liaison fluidique avec la lumière de corps de base (31), **caractérisé en ce que** l'au moins un corps latéral (32 ; 56) partant du corps de base (44 ; 52), dans l'état auto-expansé, est sorti suivant un certain angle par rapport à la direction longitudinale du corps de base (44 ; 52), l'angle étant formé par une sortie coudée préformée par rapport à la direction longitudinale du corps de base (44, 52) d'au moins une portion courbée en pointe (30 ; 58) d'un ressort d'endoprothèse (14 ; 61) du corps de base (44, 52) dans l'état auto-expansé.

2. Implant vasculaire (10 ; 50) selon la revendication 1, **caractérisé en ce qu'**une portion courbée en pointe (20 ; 30 ; 57 ; 58) est à chaque fois formée de deux branches (23, 24 ; 26 ; 27) et d'un sommet (24a, 24b, 24c) ou d'un point le plus bas (25a, 25b, 25c) situé entre les branches (23, 24 ; 26, 27).

3. Implant vasculaire (10 ; 50) selon la revendication 1 ou 2, **caractérisé en ce que** l'angle est compris entre 20° et 90°, et en particulier est compris entre 35° et 55°, de préférence vaut 45°.

4. Implant vasculaire (10 ; 50) selon l'une quelconque des revendications précédentes, **caractérisé en ce que** l'au moins un corps latéral (32 ; 56) présente une première portion de corps latéral (75) avec au moins un ressort d'endoprothèse de corps latéral (34 ; 72) enroulé en forme de méandres et un matériau d'implant de corps latéral (36 ; 71) fixé au ressort d'endoprothèse de corps latéral (34 ; 72) et au ressort d'endoprothèse (14 ; 61), sorti de manière coudée, du corps de base (44 ; 52) et reliant ceux-ci, l'au moins un ressort d'endoprothèse de corps latéral (34 ; 72) et le ressort d'endoprothèse (14 ; 61), sorti de manière coudée, du corps de base (44 ; 52), étant connectés seulement par le biais du matériau d'implant de corps latéral (36 ; 71) et n'étant pas connectés l'un à l'autre.

5. Implant vasculaire (10 ; 50) selon l'une quelconque des revendications précédentes, **caractérisé en ce que** le corps de base (44 ; 52) et/ou l'au moins un corps latéral (32 ; 56) présentent en outre de plus au moins une portion supplémentaire (76) qui ne présente pas de ressorts d'endoprothèse (12, 13, 14, 15, 16, 17, 18, 19 ; 59, 60, 61, 62, 63, 46, 65, 66), ou qui présente une endoprothèse tressée ou produite par laser (77), l'au moins une portion supplémentaire (76) se raccordant proximalement ou distalement à la portion (28 ; 69 ; 75).

6. Implant vasculaire (10 ; 50) selon l'une quelconque des revendications précédentes 2 à 5, **caractérisé en ce qu'**au moins trois branches (22, 23, 26), qui relient un premier sommet (24a), un premier point le plus bas (25a) suivant le premier sommet (24a) dans la direction périphérique, un deuxième sommet (24b) suivant le premier point le plus bas (25a) dans la direction périphérique et un deuxième point le plus bas (25b) suivant le deuxième sommet (24b) dans la direction périphérique, présentent à chaque fois des longueurs différentes de sorte que l'on obtienne une allure en forme de méandres d'un ressort d'endoprothèse (12, 13, 14, 15, 16, 17, 18, 19 ; 59, 60, 61, 62, 63, 46, 65, 66) avec des portions courbées en pointes irrégulières (20 ; 57).

7. Implant vasculaire (10 ; 50) selon l'une quelconque des revendications 1 à 6, **caractérisé en ce qu'**au moins deux ressorts d'endoprothèse (12, 13, 14, 15, 16, 17, 18, 19 ; 59, 60, 61, 62, 63, 46, 65, 66) disposés l'un derrière l'autre dans la direction longitudinale sont disposés par rapport à la direction longitudinale du corps de base cylindrique creux (44 ; 52) et l'un par rapport à l'autre de telle sorte que les sommets (24a, 24b, 24c) des portions courbées en pointe (20 ; 57) d'un premier ressort d'endoprothèse (12, 13, 14, 15, 16, 17, 18, 19 ; 59, 60, 61, 62, 63, 46, 65, 66) soient en regard de points les plus bas respectifs (25a, 25b, 25c) de portions courbées en pointe (20 ; 57) d'un deuxième ressort d'endoprothèse (12, 13, 14, 15, 16, 17, 18, 19 ; 59, 60, 61, 62, 63, 46, 65, 66) disposé en aval dans la direction longitudinale de l'implant vasculaire (10 ; 50) à distance les uns des autres et suivant une ligne qui est parallèle à la direction longitudinale de l'implant vasculaire.

8. Implant vasculaire (10 ; 50) selon l'une quelconque des revendications 2 à 5, **caractérisé en ce qu'**au moins deux ressorts d'endoprothèse (12, 13, 14, 15, 16, 17, 18, 19 ; 59, 60, 61, 62, 63, 46, 65, 66) disposés l'un derrière l'autre dans la direction longitudinale présentent à chaque fois des branches (23, 24 ; 26, 27) avec des longueurs différentes pour réaliser des portions courbées en pointe (20 ; 30 ; 57 ; 58) de hauteurs différentes, se suivant sur la périphérie, avec des portions courbées en pointe plus hautes (24a, 24c ; 25a, 25c) et plus courtes (24b ; 25b), et **en ce qu'**une portion courbée en pointe (24a, 24c) plus haute tournée dans la direction proximale x d'un premier ressort d'endoprothèse (19) est opposée à une portion courbée en pointe plus courte (25b) tournée dans la direction distale y d'un deuxième ressort d'endoprothèse (18) disposé en aval proximalement à une certaine distance et suivant une ligne imaginaire qui est parallèle à la direction longitudinale de l'implant vasculaire (10), et/ou **en ce qu'**une portion courbée en pointe plus courte (24b) tournée dans la direction proximale x d'un premier ressort d'endoprothèse (19) est opposée à une portion courbée en pointe plus longue (25a) tournée dans la direction distale y d'un deuxième ressort d'endoprothèse (18) disposé en aval dans la direction proximale x à une certaine distance et suivant une ligne imaginaire (90) qui est parallèle à la direction longitudinale de l'implant vasculaire (10 ; 50).

9. Implant vasculaire (10 ; 50) selon l'une quelconque des revendications 1 à 8, **caractérisé en ce que** le matériau d'implant de corps latéral (36 ; 71) est connecté, de préférence cousu, au matériau d'implant (43 ; 70) du corps de base (44 ; 52).

10. Implant vasculaire (10 ; 50) selon l'une quelconque des revendications 1 à 9, **caractérisé en ce que** les ressorts d'endoprothèse (12, 13, 14, 15, 16, 17, 18, 19 ; 59, 60, 61, 62, 63, 46, 65, 66) du corps de base (44 ; 52) et/ou du corps latéral (32 ; 56) sont montés à l'intérieur contre, ou à l'extérieur sur, le matériau d'implant (43 ; 70 ; 36 ; 71) du corps de base cylindrique creux (44 ; 52) et/ou du corps latéral (32 ; 56).

11. Implant vasculaire (10 ; 50) selon l'une quelconque des revendications 1 à 10, **caractérisé en ce qu'**il est prévu entre un et quatre corps latéraux (32 ; 56) sortis suivant un certain angle par rapport au corps de base (44 ; 52).

12. Procédé de fabrication d'un implant vasculaire (10 ; 50) selon l'une quelconque des revendications 1 à 11, **caractérisé en ce qu'**il présente les étapes suivantes :
- fourniture d'une pièce moulée (80) avec un corps de base de forme cylindrique (82) et avec un élément de pièce moulée (84) faisant saillie suivant un certain angle par rapport au corps de base de forme cylindrique (82) et connecté fixement à la pièce moulée (80), lequel présente une surface oblique arrondie (86), la pièce moulée (80) présentant en outre des éléments en forme de goupille (88) dans la position de sommets et de points les plus bas de ressorts d'endoprothèse s'enroulant en forme de méandres,
- fourniture d'un fil formant un ressort d'endoprothèse, de préférence en nitinol,
- enroulement du fil formant le ressort d'endoprothèse par traction sur la pièce moulée (80) et par le biais des éléments en forme de goupille (88), de telle sorte que les ressorts d'endoprothèse s'enroulant forme de méandres (12, 13, 14, 15, 16, 17, 18, 19 ; 59, 60, 61, 62, 63, 46, 65, 66) soient enroulés avec des portions courbées en pointe (20 ; 30) autour de la pièce moulée (80) et soient fixés sur la pièce moulée (80), l'au moins une portion courbée en pointe sortie angulairement (30 ; 58) étant réalisée sur l'élément de pièce moulée saillant angulairement (84).
